(19) Europäisches Patentamt
European Patent Office
Office européen des brevets

(11) Numéro de publication : **0 201 386 B1**

(12) # FASCICULE DE BREVET EUROPÉEN

(45) Date de publication du fascicule du brevet : **27.07.88**

(51) Int. Cl.[4] : **C 07 C127/15**

(21) Numéro de dépôt : **86400798.4**

(22) Date de dépôt : **15.04.86**

(54) **Nouveaux dérivés acryliques de l'urée.**

(30) Priorité : **23.04.85 FR 8506106**

(43) Date de publication de la demande : **17.12.86 Bulletin 86/46**

(45) Mention de la délivrance du brevet : **27.07.88 Bulletin 88/30**

(84) Etats contractants désignés : **AT BE CH DE GB IT LI LU NL SE**

(56) Documents cités : **CH-A- 316 404**

(73) Titulaire : **NORSOLOR S.A.**
**Tour Aurore Place des Reflets**
**F-92080 Paris la Defense Cédex 5 (FR)**

(72) Inventeur : **Lalo, Jack**
**21, rue de Digue Appt. 128**
**F-31300 Toulouse (FR)**

(74) Mandataire : **Rieux, Michel**
**NORSOLOR Service Propriété Industrielle Tour Aurore Place des Reflets Cédex no. 5**
**F-92080 Paris la Défense 2 (FR)**

**EP 0 201 386 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés acryliques de l'urée et leurs procédés de préparation.

On connait déjà des dérivés acryliques de l'urée. Les composés acryliques utilisés comme produits de départ sont le plus souvent l'acrylamide et l'acrylonitrile. C'est ainsi qu'on a proposé la polycondensation de l'urée, du formol et de l'acrylamide en milieu faiblement alcalin en vue de préparer des produits méthylolés suivi de l'addition de styrène ou d'acrylate de méthyle (voir en particulier KOSTYUKOV et al — URSS 272, 550, 3 Juin 1970). On a aussi proposé de fabriquer l'acrylamido-méthylène-urée de formule :

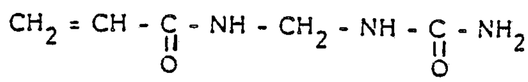

en condensant du formol, puis de l'urée en milieu sulfurique de façon à obtenir la N-méthylolurée, puis en additionnant ensuite de l'acrylonitrile de façon à obtenir l'acrylamido-méthylène-urée (voir brevet suisse 316404 de CIBA) ; les produits obtenus peuvent être polymérisés ou copolymérisés et sont utilisés comme produits auxiliaires dans l'industrie du textile, du cuir ou du papier.

La présente invention concerne de nouveaux produits de formule :

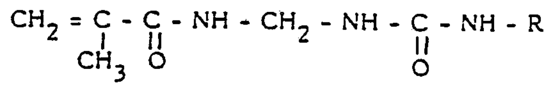

dans laquelle R est H ou R est un radical de formule

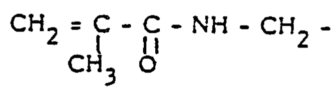

lorsque R = H le produit nouveau est le méthacrylamido-méthylène-urée,

lorsque R est

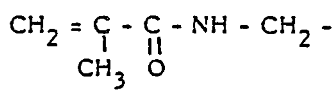

le produit est le [N,N' bis (méthacrylamido-méthylène) urée].

Les spectres infrarouge et RMN (voir figures annexées) confirment ces structures.

Le méthacrylamido-méthylène-urée a pour formule brute $C_6H_{11}N_3O_2$. Son poids moléculaire est de 157. Il se présente comme un solide blanc, son point de fusion est de 215 °C.

Le [N,N' bis (méthacrylamido-méthylène) urée] a pour formule brute $C_{11}H_{18}N_4O_3$. Son poids moléculaire est de 254. Il se présente comme un solide blanc, son point de fusion est de 239 °C.

Les nouveaux produits objet de l'invention sont polymérisables ou copolymérisables. Il conviennent particulièrement comme additifs dans les aminoplates et phénoplastes, notamment ceux utilisés comme colles à bois ; il peut aussi être utilisé comme agent réticulant.

Les nouveaux dérivés acryliques de l'urée sont préparés de la façon suivante.

Le méthacrylamido-méthylène-urée est préparé selon un mode opératoire qui comporte deux étapes. Dans la première, on prépare d'abord un dérivé méthylolé amide en faisant réagir à pH basique supérieur à 7 le méthacrylamide sur le formol. Dans la deuxième étape, on fait réagir le dérivé méthylolé amide sur l'urée à pH acide au plus égal à 4,5. La première étape est réalisée de la façon suivante. On fait réagir dans un solvant non réactif la méthacrylamide avec le formol en présence d'un catalyseur basique par exemple une amine aliphatique et de préférence en présence d'un inhibiteur éther méthylique d'hydroquinone par exemple. Ces solvants non réactifs qui conviennent pour la réalisation de cette première étape sont choisis parmi l'eau, le diméthylformamide. Lorsqu'on utilise l'eau comme solvant, le formol est introduit sous forme de solution aqueuse, la température réactionnel est voisine de 50 °C et la durée de réaction de 2 heures ; lorsqu'on utilise un solvant organique, le formol est introduit sous forme de paraformaldéhyde, la température est voisine de 80 °C et la durée de réaction de 7 heures. Pour la préparation de méthacrylamido-méthylène-urée après la mise en œuvre de la première étape on ajoute ensuite dans une seconde étape de l'urée à pH acide en quantité telle que l'on met en œuvre au moins 4 équivalents molaires d'urée pour un équivalent molaire du dérivé méthylolé amide préparé dans la première étape. La mise en œuvre de quantité inférieure à quatre équivalents conduit à la formation de [N,N' bis (méthacrylamido-méthylène) urée] comme impureté. L'addition d'urée ainsi que celle de la quantité d'acide nécessaire pour atteindre un pH au plus égal à 4,5 est faite à froid dans le milieu réactionnel. Le

mélange réactionnel est ensuite chauffé pendant environ 3 heures à une température ambiante si le solvant mis en œuvre est de l'eau, à température voisine de 80 °C, si le solvant utilisé est un solvant organique. Le produit de la réaction précipite au fur et à mesure de sa formation. Le précipité obtenu est ensuite récupéré par filtration. La préparation du [N,N' bis (méthacrylamido-méthylène) urée] est réalisée en mettant en œuvre deux étapes. La première étape est identique à celle utilisée pour la fabrication du méthacrylamido-méthylène-urée.

La deuxième étape est conduite en mettant en œuvre 0,5 équivalent molaire d'urée pour un équivalent molaire du dérivé méthylolé amide obtenu lors de la première étape.

Les exemples suivants illustrent la présente invention. Les quantités sont exprimées en partie en poids.

Exemple 1

a) 1ère étape : synthèse du dérivé méthylol-amide

Dans un réacteur agité muni d'un condenseur à reflux, on introduit la charge suivante :

24 parties de méthacrylamide
22,4 parties d'une solution de formol à 37 %
0,2 partie de triéthylamine
0,010 partie d'éther méthylique de l'hydroquinone.

Le mélange réactionnel est chauffé pendant 2 heures à 50 °C.

b) 2e étape : synthèse du méthacrylamido-méthylène-urée

Après refroidissement du mélange réactionnel à 10 °C, on ajoute :

67,6 parties d'urée
15 parties d'acide chlorhydrique concentré (50 %)

Le pH est alors de 1. Le milieu réactionnel est ensuite agité pendant 15 heures à température ambiante. On recueille un précipité qui se forme tout au long de la réaction. Après filtration, on recueille 31,2 parties de méthacrylamido-méthylène urée.

Le rendement est de 70,6 %.
Le point de fusion est de 215 °C.
L'analyse élémentaire donne les résultats suivants :

C : 45,86 % (théorie 45,85 %)
H : 7,06 % (théorie 7,05 %)
N : 26,74 % (théorie 26,86 %)

Le spectre infrarouge permet de confirmer la structure du produit obtenu (figure 1 annexée).
Le spectre RMN confirme la structure du méthacrylamido-méthylène-urée (figures 2 et 3 annexées).

Exemple 2

1ère étape

Dans le réacteur de l'exemple 1, on introduit :

200 parties de méthacrylamide
800 parties de diméthylformamide
70,5 parties de paraformaldéhyde (pureté 94 %)
4 parties de triéthylamine
0,04 partie d'éther méthylique de l'hydroquinone.

Le mélange réactionnel est chauffé pendant 7 heures à 80 °C.

2e étape

Après refroidissement du mélange réactionnel à température ambiante, on ajoute 564,5 parties d'urée et 4,3 parties d'acide sulfurique à 98 %. Le mélange réactionnel est chauffé pendant 3 heures à 80 °C : il se forme un précipité tout au long de la réaction qui est récupéré par filtration de la solution à

35 °C. Après lavage dans le diméthylformamide et dans l'acétone, on recueille 145 parties de méthacrylamido-méthylène-urée. Le rendement est de 42 %.

Le point de fusion est de 215 °C.

L'analyse élémentaire a donné les résultats suivants :

C : 45,71 %
H : 7,02 %
N : 27,07 %

Les spectres IR et RMN permettent de confirmer la structure du nouveau produit obtenu.

## Exemple 3

1ère étape

Dans le réacteur de l'exemple 1 on introduit :

85 parties de méthacrylamide
81 parties d'une solution aqueuse de formol concentrée à 37 %
1,7 partie de triéthylamine
0,01 partie d'éther méthylique de l'hydroquinone.

Le mélange réactionnel est chauffé pendant deux heures à 50 °C.

2e étape

Après refroidissement à une température inférieure à 10 °C, on ajoute 30 parties d'urée et 25 $cm^3$ d'une solution aqueuse concentrée d'acide chlorhydrique : il se forme à température ambiante un précipité, que l'on filtre, lave à l'eau et sèche à l'acétone. On recueille 105 parties de N'N'(bis méthacrylamido-méthylène) urée : le rendement est de 83 %.

Le point de fusion est de 239 °C.

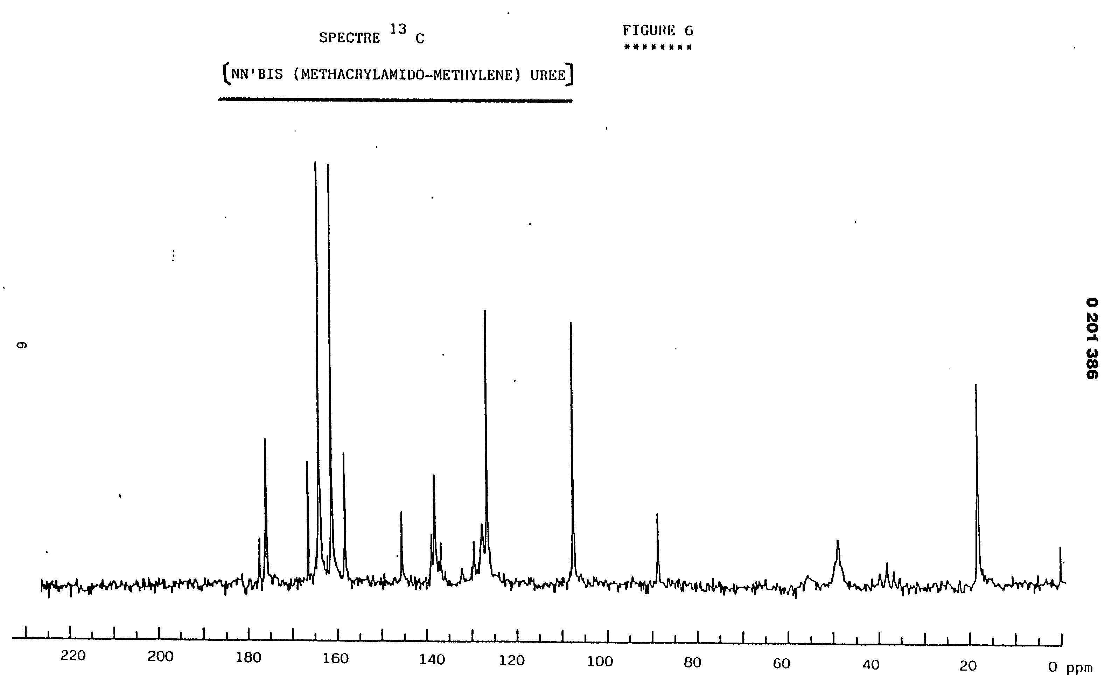

Le spectre infra-rouge et RMN permettent de confirmer la structure du nouveau produit obtenu (voir figures 4, 5, 6 annexées).

L'analyse élémentaire a donné les résultats suivants :

| | % calculés (théorie) | % trouvés |
|---|---|---|
| C | 51,97 | 51,85 |
| H | 7,09 | 7,10 |
| N | 22,05 | 22,22 |

## Exemple 4

L'exemple 1 est répété en mettant en œuvre les réactifs suivants :

42,5 parties de méthacrylamide
40,5 parties d'une solution de formol à 37 %
1 partie de triéthylamine
0,020 partie d'éther méthylique de l'hydroquinone.

Le mélange réactionnel est maintenu 2 heures à 50 °C.

Dans une deuxième étape on ajoute au mélange réactionnel préalablement refroidi à 10 °C, 60 parties d'urée puis 25 $cm^3$ d'une solution aqueuse concentrée d'acide chlorhydrique. Le mélange réactionnel est maintenu deux heures à une température de 15 °C. Il se forme un précipité au bout d'une heure qui est filtré. On obtient alors 59 parties de méthacrylamido méthylène-urée. Le rendement est de 75 %.

L'analyse par chromatographie en couche mince permet de déceler la présence d'une impureté : il s'agit du N,N'(bis-méthacrylamido-méthylène) urée.

## Exemple 5

Une résine urée-formol présente les caractéristiques suivantes :

| | |
|---|---|
| Viscosité à 20 °C | 4 poises |
| Extrait sec | 65 % |
| pH | 8 |
| $F/NH_2$ | 0,55 |

Cette résine est modifiée par 6,5 % de méthacrylamido-méthylène urée. On dispose alors d'une résine qui présente les caractéristiques suivantes : .

| | |
|---|---|
| Viscosité à 20 °C | 7,3 poises |
| Extrait sec | 65 % |
| pH | 8 |
| $F/NH_2$ | 0,55 |

Ces deux résines sont utilisées pour la fabrication de panneaux particules monocouches à partir de copeaux de bois en mettant en œuvre les mêmes conditions opératoires : les résines sont durcies à l'aide d'une solution de chlorure d'ammonium. Le tableau suivant indique les caractéristiques des panneaux obtenus :

| Type résine | Résine modifiée | Résine non modifiée |
|---|---|---|
| Résistance en traction (Kg/cm²) | 6,1 | 5,8 |
| Gonflement à l'eau V 20, % | 12,5 | 12,1 |
| Absorption, % | 69,3 | 69,6 |
| Humidité panneau % | 5,9 | 5,9 |
| Formol perforateur mg/100 g | 12,5 | 14 |

Les mesures ont été déterminées selon les normes suivantes :
Teneur en formol (perforateur) : Norme EN 120
Epaisseur, masse volumique, humidité : Norme DIN 52361
Traction V 20 : Norme DIN 68763
Gonflement (%) : Norme DIN 52364

**Revendications**

1. Nouveaux dérivés acryliques de l'urée caractérisé par le fait qu'ils ont la formule générale :

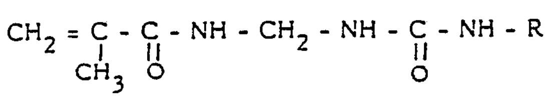

dans laquelle R est H, le dérivé étant le méthacrylamido-méthylène-urée ou R est

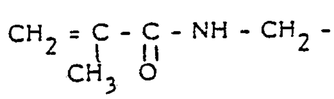

le dérivé étant le [N,N' bis (méthacrylamido-méthylène) urée]

2. Procédé de préparation du méthacrylamido-méthylène urée selon 1 caractérisé en ce qu'on fait

réagir dans une première étape dans un solvant non réactif la méthacrylamide avec le formol à pH basique supérieur à 7 et en ce que dans une deuxième étape on fait réagir le produit de la réaction obtenu dans la première étape sur l'urée à pH acide au plus égal à 4,5 la quantité d'urée mise en œuvre étant au moins égale à 4 équivalents molaires pour un équivalent molaire du dérivé méthylolé amide préparé dans la première étape.

3. Procédé de préparation de [N,N' bis (méthacrylamido-méthylène) urée] selon 1 caractérisé en ce qu'on fait réagir dans une première étape dans un solvant non réactif la méthacrylamide avec le formol à pH basique supérieur à 7 et en ce que dans une deuxième étape on fait réagir le produit de la réaction obtenu dans la première étpae sur l'urée à pH acide au plus égal à 4,5 la quantité d'urée mise en œuvre étant égale à 0,5 équivalent molaire pour un équivalent molaire du dérivé méthylolé amide obtenu dans la première étape.

4. Procédé selon 2 et 3 caractérisé en ce que le solvant non réactif est choisi parmi l'eau ou le diméthylformamide.

## Claims

1. New acrylic derivatives of urea characterized in that they have the general formula

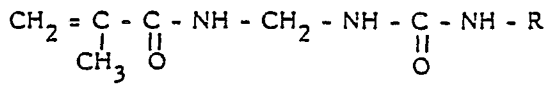

in which R is H, the derivative being methacrylamido-methyleneurea, or R is

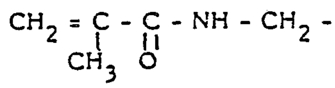

the derivative being [N,N'-bis (methacrylamidomethylene)-urea].

2. Method for preparing methacrylamidomethyleneurea according to 1, characterized in that in a first stage methacrylamide is reacted with formaldehyde at a basic pH higher than 7 in an unreactive solvent and in that, in a second stage, the reaction product obtained in the first stage is reacted with urea at an acidic pH not exceeding 4.5, the quantity of urea employed being at least equal to 4 molar equivalents for 1 molar equivalent of the methylolated amide derivative prepared in the first stage.

3. Method for preparing [N,N'-bis (methacrylamidomethylene) urea] according to 1, characterized in that in a first stage methacrylamide is reacted with formaldehyde at a basic pH higher than 7 in an unreactive solvent and in that, in a second stage, the reaction product obtained in the first stage is reacted with urea at an acidic pH not exceeding 4.5, the quantity of urea employed being equal to 0.5 molar equivalent for 1 molar equivalent of the methylolated amide derivative obtained in the first stage.

4. Process according to 2 and 3, characterized in that the unreactive solvent is chosen from water or dimethylformamide.

## Patentansprüche

1. Neue Harnstoffacrylverbindungen, gekennzeichnet durch die allgemeine Formel :

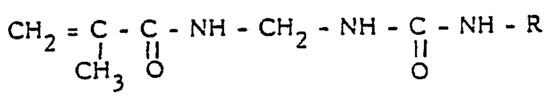

wobei, wenn R = H, die Verbindung Methacrylamidomethylenharnstoff ist, oder, wenn R

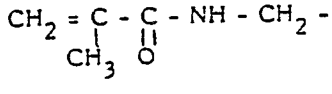

ist, die Verbindung [N,N' bis (Methacrylamidomethylen) harnstoff] ist.

2. Verfahren zur Herstellung des Methacrylamidomethylenharnstoffs nach Anspruch 1, dadurch gekennzeichnet, daß man in einem ersten Schritt in einem nicht reaktiven Lösungsmittel Methacrylamid mit Formaldehyd bei einem basischen pH-Wert über 7 reagieren läßt, und daß man in einem zweiten Schritt das im ersten Schritt erhaltene Reaktionsprodukt auf Harnstoff bei einem sauren pH-Wert von höchstens 4,5 einwirken läßt, wobei die eingesetzte Harnstoffmenge mindestens 4 Moläquivalente auf 1 Moläquivalent des im ersten Schritt erhaltenen Methylolamids beträgt.

3. Verfahren zur Herstellung von [N,N' bis (Methacrylamidomethylen) harnstoff] nach Anspruch 1, dadurch gekennzeichnet, daß man in einem ersten Schritt in einem nicht reaktiven Lösungsmittel Methacrylamid mit Formaldehyd bei einem basischen pH-Wert über 7 reagieren läßt, und daß man in einem zweiten Schritt das im ersten Schritt erhaltene Reaktionsprodukt auf Harnstoff bei einem sauren pH-Wert von höchstens 4,5 einwirken läßt, wobei die eingesetzte Harnstoffmenge 0,5 Moläquivalente auf 1 Moläquivalent des im ersten Schritt erhaltenen Methylolamids beträgt.

4. Verfahren nach Anspruch 2 oder 3, dadurch gekennzeichnet, daß das nicht reaktive Lösungsmittel Wasser oder Dimethylformamid ist.

FIGURE 1
********

METHACRYLAMIDO-METHYLENE UREE

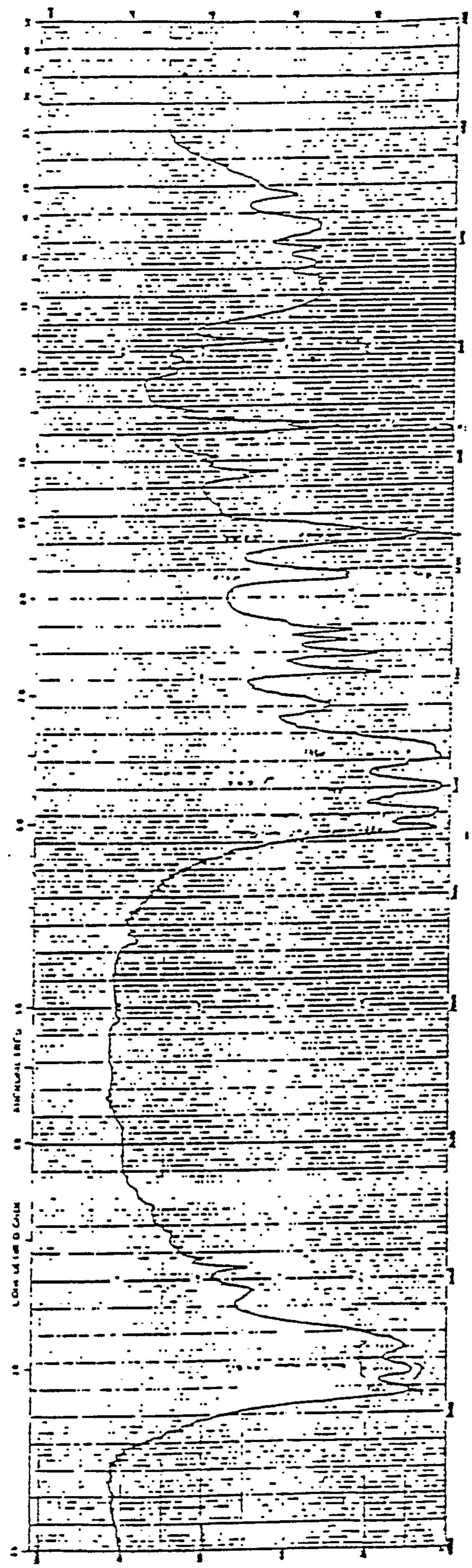

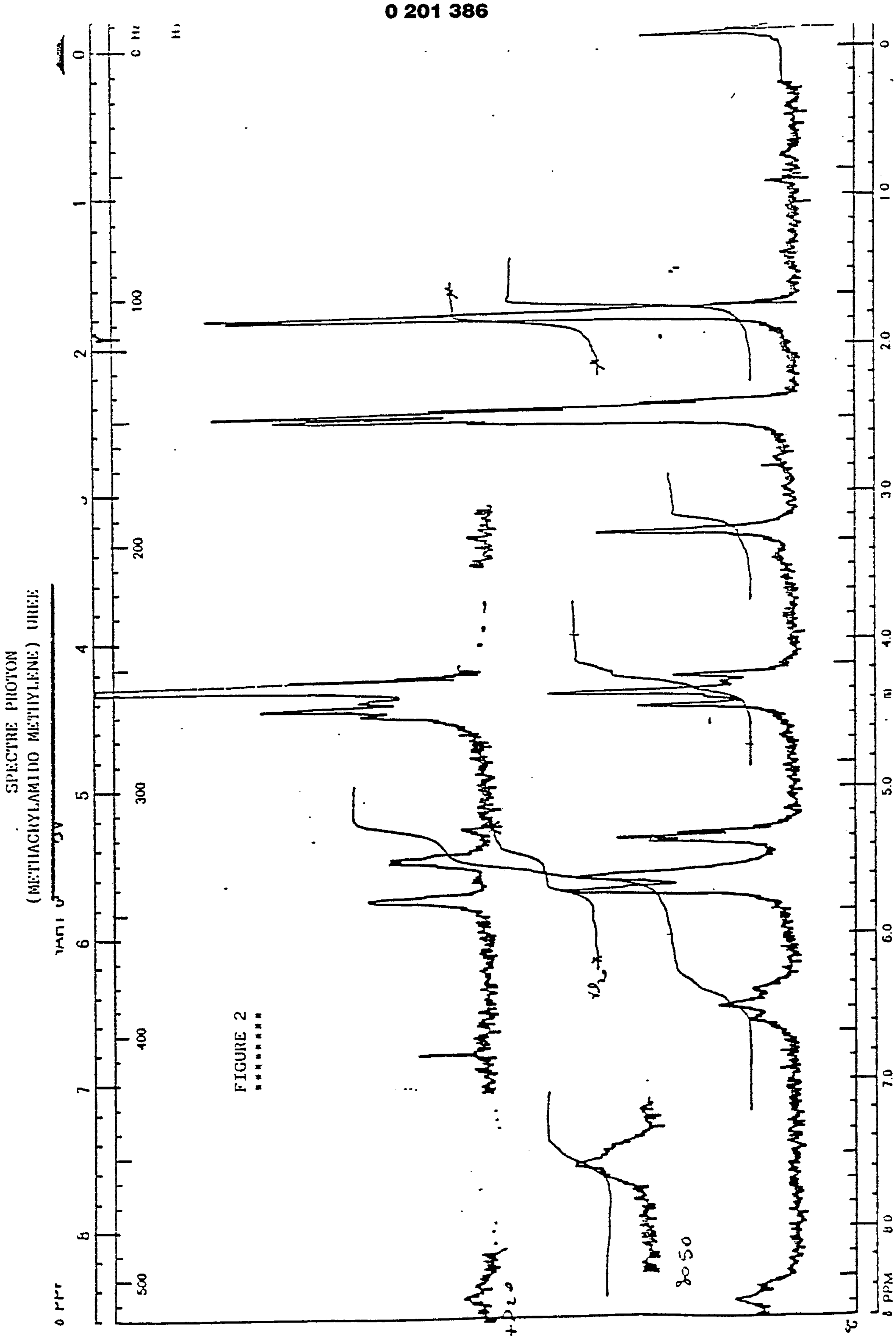
SPECTRE PROTON
(METHACRYLAMIDO METHYLENE) UREE
FIGURE 2
δ PPM

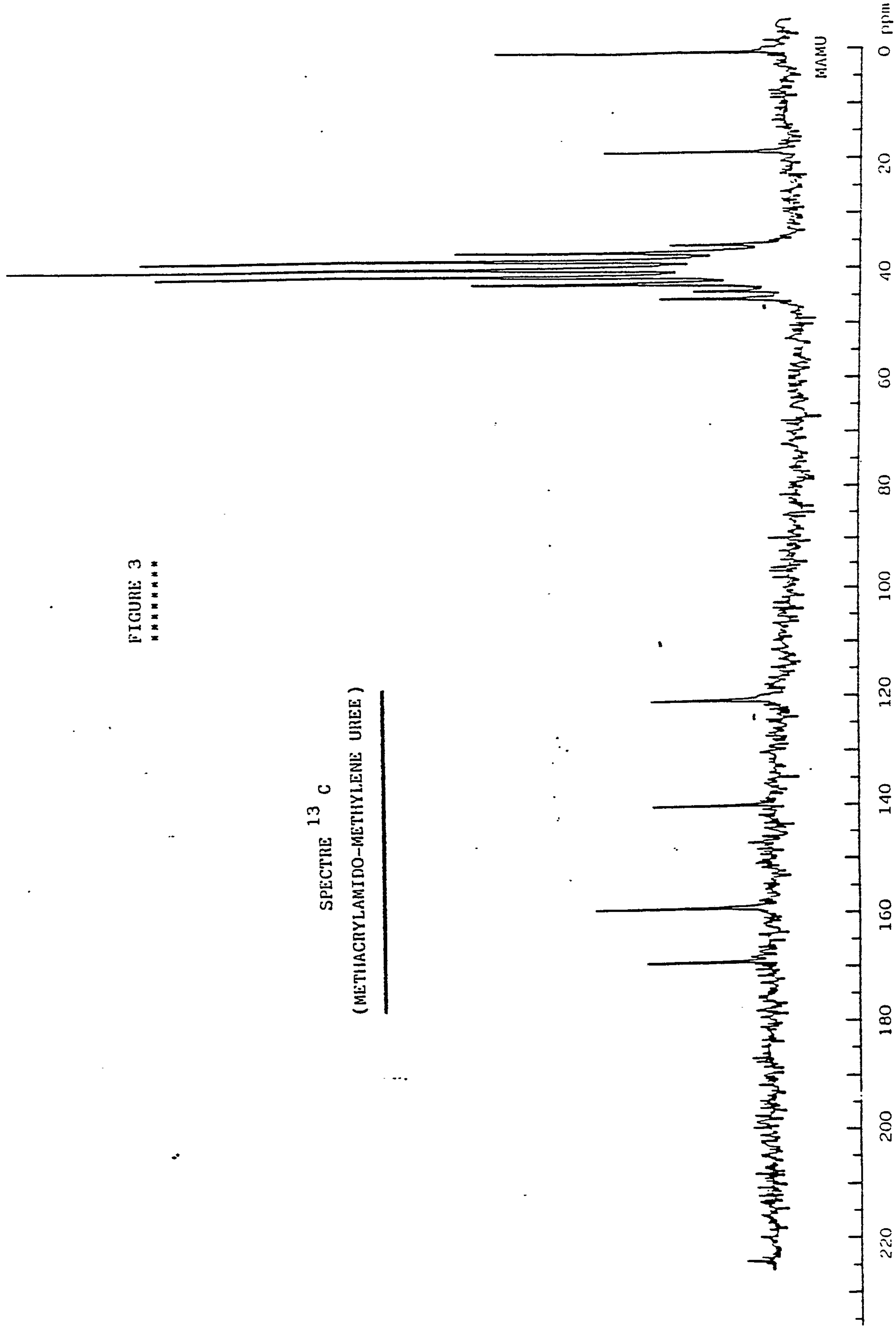


FIGURE 3
********

SPECTRE $^{13}C$
(METHACRYLAMIDO-METHYLENE UREE)

FIGURE 4

N, N' BIS (METHACRYLAMIDO METHYLENE)UREE

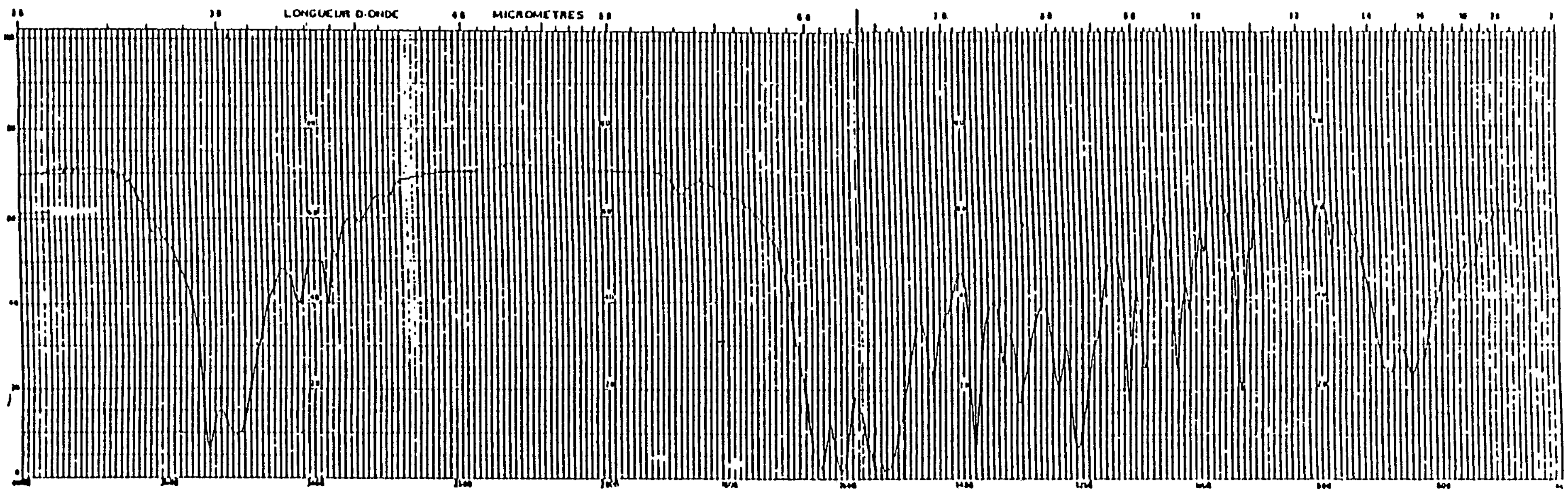

FIGURE N° 5

SPECTRE PROTON

[NN'BIS (METHACRYLAMIDO-METHYLENE) UREE]

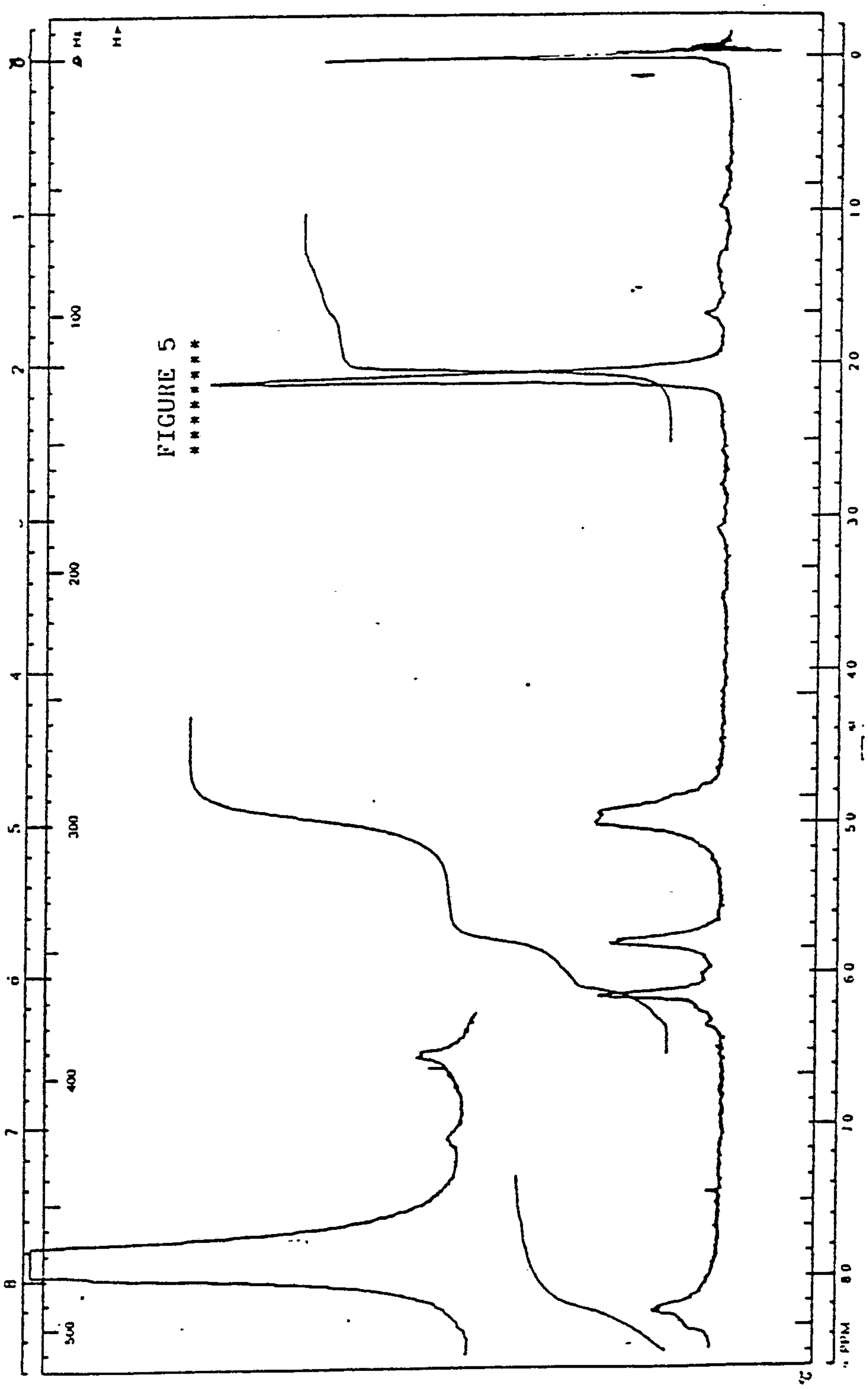

SPECTRE $^{13}C$

FIGURE 6
********

[NN'BIS (METHACRYLAMIDO-METHYLENE) UREE]